# EUROPEAN PATENT APPLICATION

(11) **EP 3 085 321 A1**
(43) Date of publication of application: **26.10.2016**
(21) Application number: 16162117.2
(22) Date of filing: 24.03.2016
(51) Int. Cl.: A61B 17/88, A61B 17/17, A61B 17/70, A61B 17/90, A61B 90/00, A61F 2/30, A61F 2/46, A61B 17/86

(54) **CANNULATED COUNTERSINK AND DEPTH GAUGE MULTI-INSTRUMENT FOR DETERMINING REQUIRED SCREW LENGTH**

(30) Priority: 24.03.2015 US 201562137471 P
(71) Applicant: Flower Orthopedics Corporation, Horsham, PA 19044 (US)
(72) Inventor: GARVEY, Brian, Media, PA 19063 (US); BURCKHARDT, Oliver, Philadelphia, PA 19119 (US)
(74) Representative: Schiweck, Weinzierl & Koch

(57) **Abstract**

The present invention relates to a multi-tool or multi-purpose device (100) comprising a countersink (50) and a depth gauge (40, 80, 90, 60). The device comprises a cannula (40) for insertion of a guide wire (60). The cannula can have a cutaway portion (80) with a graduated scale (90). The countersink can comprise cutting flutes (51).The countersink depth gauge multi-tool device can be used in a method of determining the necessary length of a screw to be implanted into bone. Also provided herein are sterile kits comprising single-use instrumentation for use in orthopedic procedures.

## Description

### CROSS-REFERENCE TO RELATED APPLICATIONS

This application claims priority to U.S. Provisional Patent Application No. 62/137,471 filed March 24, 2015, the contents of which are incorporated by reference herein in its entirety.

### BACKGROUND OF THE INVENTION

During certain orthopedic procedures, it is necessary to use a countersink to prepare the bone for the seating of a screw. Further, in certain instances, it is desired for the surgeon to know the required length of the screw to be inserted into the bone. Traditionally, these two steps are performed by separate devices, which increases the risk of adverse effects as each device is separately applied and removed.

Thus, there is a need in the art for improved devices for orthopedic procedures. The present invention satisfies this unmet need.

### SUMMARY OF THE INVENTION

In one aspect the present invention provide a multi-purpose device for use in an orthopedic procedure. The device comprises an elongated body having a proximal end and a distal end and a length therebetween; a countersink positioned at the distal end; and a cannula extending from an opening in the distal end along at least a portion of the length of the elongated body. At least a portion of the cannula that is proximal to the countersink is visible through the outer surface of the elongated body.

In one embodiment, the countersink comprises one or more cutting flutes. In one embodiment, the cannula of the device has a diameter sized to receive a guide wire.

In one embodiment, the device comprises at least one graduation marking along the elongated body where at least a portion of the cannula is visible from the outer surface.

In another aspect, the present invention provides a system for use in an orthopedic procedure. The system comprises one or more guide wires and a tool having an elongated body terminating at a proximal end and a distal end, where the distal end includes at least one fluted cutting edge and an opening that leads into a cannula through an interior region of the elongated body. At least a portion of the cannula is visible from the outer surface of the elongated body in a cutaway region. The guide wire is sized to pass a terminal end of the guide wire through the distal opening and into the cannula of the tool, such that at least a portion of the guide wire is visible in the cutaway region.

In one embodiment, the tool comprises at least one graduation marking along the elongated body where at least a portion of the cannula is visible from the outer surface. In one embodiment, the guide wire has at least one marking along its length, such that the at least one marking is identifiable when positioned in the cutaway region of the tool.

In one aspect, the present invention provides a method of determining the length of a screw to be inserted into a bone during an orthopedic procedure using the multi-purpose device and a guide wire having a predetermined length. The method comprises inserting the distal end of a guide wire having a predetermined length into bone, such that at least a portion of the proximal end of the guide wire extends outward from the bone surface. The method comprises advancing the proximal end of the guide wire through the distal end opening of the multi-purpose device described elsewhere herein, such that the guide wire is inserted into at least a portion of the cannula. The method comprises positioning the distal end of the device in contact with the bone surface and observing the location of proximal end of the guide wire within the exposed region of the cannula of the device. The method comprises measuring the length of the guide wire within the cannula and calculating the length of the guide wire inserted into bone based on the difference between the predetermined length of the guide wire and the measured length of the guide wire within the cannula. The method comprises determining the length of the screw based on the calculated length of the guide wire inserted into the bone.

In one embodiment the device comprises at least one graduation marking along the elongated body where at least a portion of the cannula is visible from the outer surface. In one embodiment, measuring the length of the guide wire within the cannula comprises identifying a graduation marking closest to the proximal end of the guide wire.

In one embodiment, the necessary length of the screw is equal to the calculated length of the guide wire inserted into the bone. In one embodiment, the necessary length of the screw is equal to the calculated length of the guide wire minus the predetermined length of a region at the distal end of the guide wire.

In one aspect, the present invention provides a method of determining the length of a screw to be inserted into a bone during an orthopedic procedure using the multi-purpose tool and a guide wire having at least one marking. The method comprises inserting the distal end of a guide wire having at least one marking along its length into bone, such that at least a portion of the proximal end of the guide wire extends outward from the bone surface, wherein the at least one marking of the guide wire is indicative of the length of the guide wire from its distal end to the at least one marking. The method comprises advancing the proximal end of the guide wire through the distal end opening of the multi-purpose device, such that the guide wire is inserted into at least a portion of the cannula. The method comprises positioning the distal end of the device in contact with the bone surface and observing the location of the at least one marking of the guide wire within the exposed region of the cannula of the device. The method comprises measuring the length of the guide wire within the cannula, and calculating the length of the guide wire inserted into bone based on the difference between the known length from the distal end of the guide wire to the at least one marking and the measured length of the guide wire within the cannula. The method comprises determining the length of the screw based on the calculated length of the guide wire inserted into the bone.

In one embodiment, the device comprises at least one graduation marking along the elongated body where at least a portion of the cannula is visible from the outer surface, and measuring the length of the guide wire within the cannula comprises identifying a graduation marking closest to the at least one marking of the guide wire.

In one aspect, the present invention provides a sterile kit comprising the multi-purpose device described herein. In one embodiment, the kit further comprises one or more implantable screws, implantable bone plates, plating screws, screwdrivers, screw depth gauges, preliminary fixation devices, guide wires, drill bits, and/or drill guides. In one embodiment, the one or more instruments are individually packaged in sterile packages. In one embodiment, the kit comprises instructional material.

In one aspect, the present invention provides a method of providing a kit for use in a surgical procedure. The method comprises receiving a request for one or more instruments for use in a surgical procedure; processing the one or more requested instruments; packaging the one or more processed instruments into one or more sterile packages to form a sterile kit; and providing the sterile kit to a user. In one embodiment, processing the one or more requested instruments comprises sterilizing the one or more requested instruments.

In one embodiment, the one or more instruments comprise the multi-purpose device described herein. In one embodiment, the one or more instruments comprise the multi-purpose device described herein and one or more guide wires.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.
Figure 1 is a schematic illustrating an exemplary device of the invention.
Figure 2 is a schematic illustrating an exemplary use of the device shown in Figure 1.
Figure 3 is a schematic illustrating an exemplary kit of the invention.

### DETAILED DESCRIPTION

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

As used herein, each of the following terms has the meaning associated with it in this section.

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20%, ±10%, ±5%, ±1%, or ±0.1% from the specified value, as such variations are appropriate to perform the disclosed methods.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The present invention relates to a multi-tool device comprising a countersink and a depth gauge. The device may be used in a variety of orthopedic surgical procedures, including but not limited to, procedures to repair a broken bone or the fusion of two or more bones together.

In one aspect, the device comprises a countersink, which may be used to prepare a target site on a bone for the subsequent seating of a screw. For example, the countersink of the device is used to provide a countersunk recess in the bone, such that the head of the screw upon insertion, either sits below the bone surface, is flush with the bone surface, or is at least partially embedded in the bone surface.

In one aspect, the device comprises a depth gauge to determine the necessary length of a screw to be inserted at the target site. For example, in certain embodiments, the device is used to measure the depth to which a guide wire is inserted into the target site, which thereby provides a measurement of the necessary screw length.

In certain embodiments, the device is provided in a sterile package. For example, the sterile package may comprise one or more of the devices described herein. In certain embodiments, the sterile package comprises one or more of the devices described herein, together with one or more instruments that may be necessary to perform the desired orthopedic procedure. The sterile package allows for immediate use of the device and other instruments without the need for pre-processing. In certain aspects, the device and other instruments are configured for single-use applications.

### Device

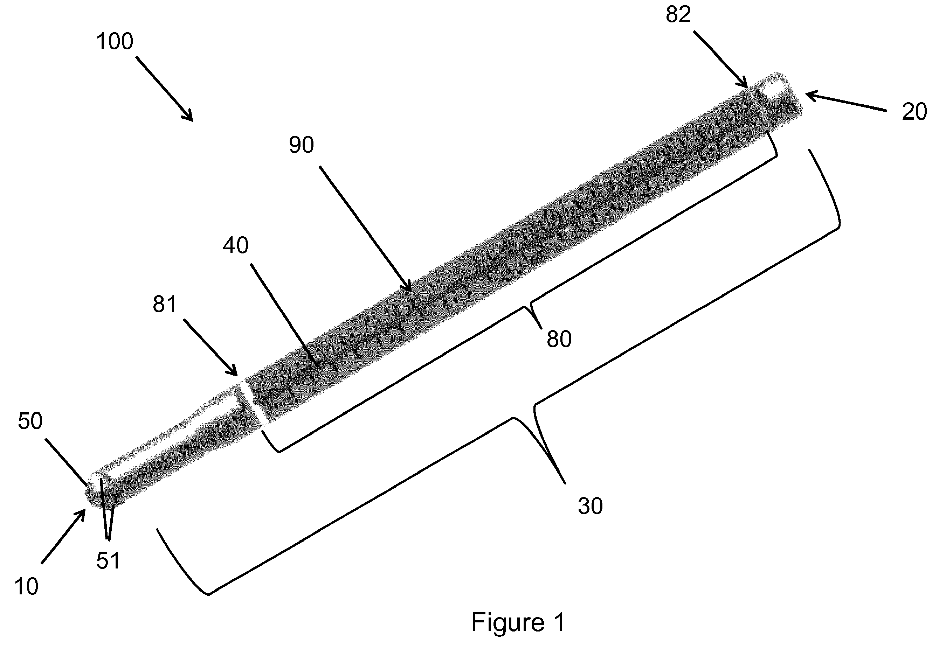

Figure 1 depicts an exemplary device 100 of the present invention. Device 100 comprises an elongated body 30 having a distal end 10 and a proximal end 20. In certain embodiments, device 100 may have a total length of about 10mm to about 1000mm. For example, in certain embodiments, device 100 has a total length of about 10mm, 20mm, 30mm, 40mm, 50mm, 60mm, 70mm, 80mm, 90mm, 100mm, 200mm, 250mm, 300mm, 400mm, 500mm, 600mm, 700mm, 800mm, 900mm, or 1000mm. Device 100 may be manufactured from any suitable material such as any biocompatible or non-bioreactive materials, including but not limited to stainless steel, aluminum plastics, and the like.

Distal end 10 of device 100 comprises a countersink 50, which includes one or more cutting flutes 51 used to prepare the bone for the subsequent seating of a screw. Cutting flutes 51 may have a curved cross-sectional shape, a circular cross-sectional shape, or a semi-circular cross-section shape, as would be understood by those skilled in the art for generating a recess into the bone surface. In one embodiment, cutting flutes 51 are made of a metal or other hard, rigid material that is strong enough for the leading edge to cut, scrape away, or otherwise remove the portion of the bone when device 100 is actuated. As contemplated herein, device 100 may be actuated by rotational movement, with or without forward pressure, thereby causing cutting flutes 51 to engage and remove a portion of the bone to form the desired recess. As contemplated herein, countersink 50 may be used to form an appropriately sized and shaped recess in the bone to allow for a subsequently inserted screw head to be at least partially embedded in the bone, be flush with or sit below the bone surface.

In certain embodiments, countersink 50 has a tip geometry that accommodates the particular geometry of the screw head to be used. For example, in certain embodiments, the geometry of countersink 50 is designed to produce a recess with a defined angle to match the angle of the screw head being used. However, the geometry of countersink 50 may or may not be identical to the undersurface of the screw head. Further, countersink 50 is appropriate for accommodating the insertion of various screw heads with different head diameters.

In one embodiment, device 100 comprises a cannula or channel 40 running from an opening in distal end 10 through at least a portion of the length of device 100. For example, in certain embodiments, cannula 40 runs from the opening in distal end 10 through elongated body 30 of device 100 to proximal end 20 of device 100. In other embodiments, cannula 40 may extend from the opening in distal end 10 up to 10%, 20%, 30%, 40%, 50%, 60%, 70%, 80%, 90%, or 100% of the length of device 100. In still other embodiments, device 100 may include an opening at proximal end 20, such that cannula 40 is a channel running the entire length of device 100 and accessible from both the distal and proximal end openings. Cannula 40 may have a diameter that is greater than a standard guide wire, such that a guide wire extending from the target bone surface can be inserted into the distal end 10 opening and pass at least partially through, or all the way through, the length of cannula 40.

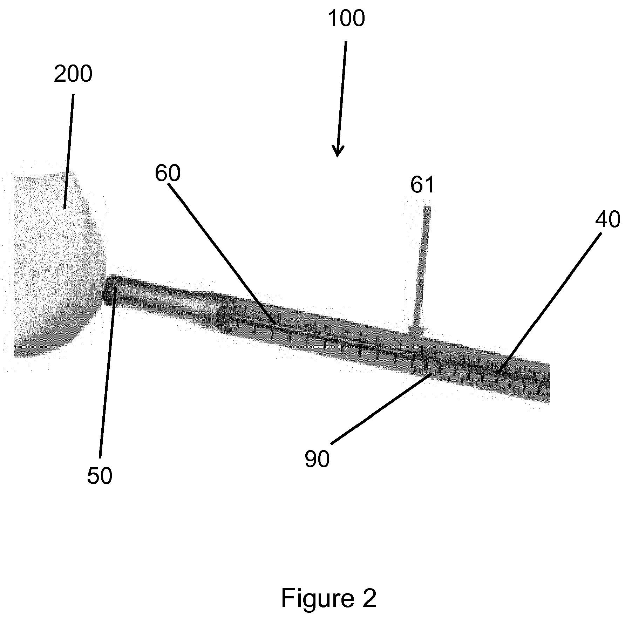

As mentioned previously, device 100 may be used for measurement of the screw length needed for a desired application. For example, as shown in Figures 1 and 2, elongated body 30 of device 100 may include a cutaway portion 80 at least partially along its length. Cutaway portion 80 may be defined by a distal boundary 81 and a proximal boundary 82, in between which cannula 40 may be at least partially exposed to allow for visual observation of a guide wire 60 and terminal end 61 of guide wire 60 positioned within cannula 40. Further, cutaway portion 80 may span 5%, 10%, 20%, 30%, 40%, 50%, 60%, 70%, 80%, or 90% of the length of device 100. In one embodiment, cutaway portion 80 is a notch to allow for visualization of cannula 40 in only a small area. Cutaway portion 80 may be positioned anywhere along the length of device 100, provided it does not interfere with the structural requirements of countersink 50. Further, the partially exposed, visual portion of cannula 40 within cutaway portion 80 may be recessed far enough into the elongated shaft or shank of device 100, such that linear movement of the guide wire 60 within cannula 40 cannot be interfered with.

In one embodiment, cutaway portion 80 comprises measurement graduations, such as a numbering scale 90, to allow for measurement of the guide wire extending from the bone surface. As contemplated herein, this measurement of the guide wire may be used to determine the necessary screw length for subsequent insertion into the bone. In one embodiment, numbering scale 90 provides the length of the guide wire from the distal end opening of device 100 to the terminal end 61 of the guide wire. In another embodiment, numbering scale 90 provides the length of the guide wire from the proximal end of countersink 50 to the terminal end 61 of the guide wire.

For example, in one embodiment, the guide wire has a predetermined length, and numbering scale 90 provides a measurement of the length of the guide wire external from the bone, thereby allowing determination of the length of the guide wire inserted into the bone, and thus providing the desired length of the screw for insertion. In another embodiment, numbering scale 90 provides a direct measurement of the depth of the inserted guide wire. For example, device 100 can be used with a predefined length of guide wire or a guide wire having a visible marking (e.g., laser marking, color marking, groove, bump, etc.) indicative of length, and thus numbering scale 90 of device 100 can provide a direct indication of the depth of the inserted guide wire. In certain embodiments, the guide wire has a plurality of visible markings, where each marking is indicative of a length of the guide wire. Numbering scale 90 may have markings in millimeters, centimeters, inches, arbitrary units, or any other suitable graduation for determining length measurement or positioning.

In certain embodiments, elongated body 30 comprises one or more handling features, such as flats or knurling, that allow for ergonomic use of device 100. For example, the one or more features may allow for ergonomic handing of device 100 and rotation of device 100, for the actuation of countersink 50.

### Methods

The present invention also provides a method for guiding placement of a countersunk recess in a target site of bone. In certain embodiments, the method comprises inserting guide wire 60 into a target site of bone 200, where the target site is the site in which a screw will eventually be inserted during a procedure. Guide wire 60 may be a standard, stiff, rigid metal wire or steel wire as commonly used by those skilled in the art. For example, guide wire 60 may be made of a metallic material that is strong enough to penetrate the surface of bone 200 and the interior of bone 200. Guide wire 60 can be configured to be drilled, screwed, or twisted into bone 200 using standard techniques understood in the art.

Once guide wire 60 is inserted into the target site of bone 200, the opening in distal end 10 leading into cannula 40 of device 100 is placed over guide wire 60 such that guide wire 60 extends into the interior of device 100. In certain embodiments, device 100 is advanced over guide wire 60 until countersink 50 of device 100 touches the surface of bone 200. In certain embodiments, device 100 is actuated to produce a countersunk recess in bone 200, thereby preparing the bone for the subsequent seating of screw to sit flush, below the bone surface, or otherwise be at least partially embedded into the bone surface. For example, after guide wire 60 has been inserted into cannula 40, cutting flutes 51 of countersink 50 can be placed in direct contact with the target site of bone 200 as guided by guide wire 60. Once in contact with the surface of bone 200, device 100 is actuated, for example by rotating, turning, or twisting, thereby allowing cutting flutes 51 to form the desired shape and depth recess to produce a countersunk recess for the head of a screw. It should be appreciated that accurate measurement of necessary screw length may or may not be predicated on the use of countersink 50. It should be appreciated that a cannulated drill may or may not be used over guide wire 60 to create a pilot hole in bone 200 before or after the use of countersink 50, and before the placement of the screw.

The present invention also provides a method of measuring the length of a screw required for a desired surgical application. For example, as shown in Figure 2, the method utilizes device 100 for measuring the depth that a guide wire 60 is inserted into a target site of a bone 200. As a result of measuring the depth of guide wire 60, a proper screw length may be determined for subsequent insertion. In certain embodiments, the method utilizes device 100 with a guide wire 60 having a predefined length. In certain embodiments, the method utilizes device 100 herein with a guide wire 60 having one or more visible markings along its length, where each marking is indicative of length.

In one embodiment, the length of guide wire 60 external to bone 200 and/or length of guide wire 60 inserted into bone 200 can be read from the terminal end 61 of guide wire, 60 as visualized in the exposed region of cannula 40. In one embodiment, the length of guide wire 60 external to bone 200 and/or length of guide wire 60 inserted into bone 200 can be read from one or more visible markings on guide wire 60.

For example, in one embodiment, numbering scale 90 has a plurality of graduation markings indicative of the length of the screw required, as measured from the terminal end 61 of the guide wire 60 or from one or more markings along the guide wire 60. For example, if guide wire 60 has a predetermined length of X (or if the single marking of guide wire 60 is at length X from the distal tip of guide wire 60), numbering scale 90 can be used to measure the length Y from the surface of bone 200 to proximal tip 61 of guide wire 60 (or to the single marking). The necessary screw length can then be calculated as X-Y. In another embodiment, device 100 may be configured to be used with a guide wire 60 of predetermined length X (or with a guide wire having a marking at length X), and thus numbering scale 90 can be preconfigured to read out a value of X-Y.

In another embodiment, guide wire 60 comprises a plurality of markings indicative of length, and numbering scale 90 includes a single marking at a defined distance away from distal end 10 of device 100. Thus, the length of the screw can be read from the markings of guide wire. For example, if numbering scale 90 of device 100 comprises a single marking at a length A from distal end 10, the markings of guide wire 60 can be used to determine the length B from the distal tip of guide wire 60 to the single marking of numbering scale 90 of device 100. The necessary screw length can then be calculated as B-A.

In certain aspects, the necessary screw length may not measure to the distal tip of guide wire 60. For example, the measurement may be taken from a predetermined amount less than or greater than the distance to the distal tip of guide wire 60.

In certain aspects, guide wire 60 may comprise a region of threading, ribbing, or grooves at its distal end, which may or may not be related to the length of screw required. Thus, in certain embodiments, the measurement of necessary screw length may be made to disregard such region. In other embodiments, the measurement is made to account for such a region.

### Kits

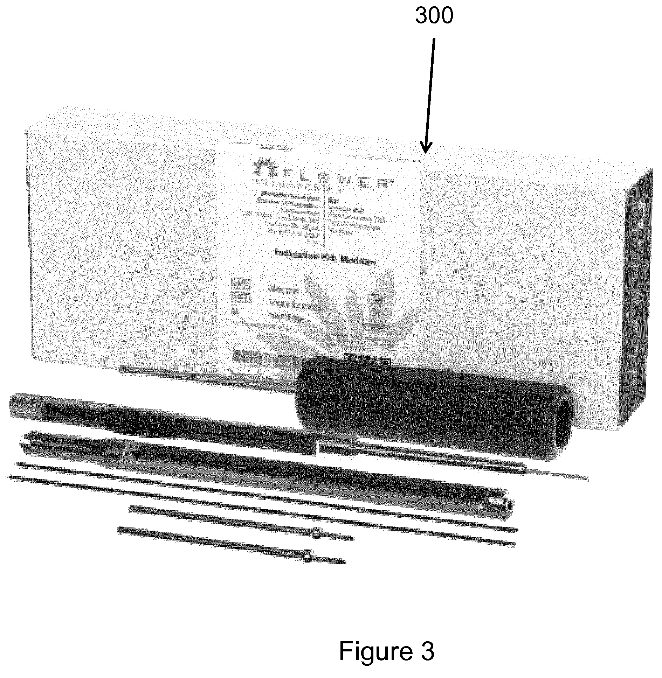

As depicted in Figure 3, in one aspect, the present invention provides an implant kit 300 comprising instrumentation to place a bone implant during an orthopedic procedure. In certain embodiments, implant kit 300 is a sterile packaged kit. In certain embodiments, the one or more instruments of implant kit 300 are sterile and contained in one or more individual sterile packages within kit 300. The sterile implant kit described herein is thus immediately ready for surgical application upon removal of the instruments from their respective packages without the need for pre-operation cleaning, sterilizing, or other processing. In certain aspects, the one or more instruments of implant kit 300 are single-use instruments. For example, in one embodiment, the one or more instruments of implant kit 300 are sterile and disposable. In another embodiment, the one or more instruments of implant kit 300 are repackaged after use, where, in certain embodiments, the one or more instruments may be reprocessed for future use. In one embodiment, the instruments may be provided in one or more blister packaging. Each blister may comprise a plastic container (e.g., PETG) component and a lid (e.g., Tyvek) component.

In certain embodiments, implant kit 300 comprises a countersink-depth gauge multi-tool device, such as device 100 of Figures 1 and 2. In certain embodiments, implant kit 300 may optionally include other instruments, such as and without limitation, one or more implantable screws, one or more implantable bone plates, one or more plating screws, one or more screw drivers, one or more screw depth gauges, one or more preliminary fixation devices, one or more guide wires, one or more drill bits, and/or one or more drill guides. For example, in one embodiment, implant kit 300 comprises one or more implantable screws, which may be cannulated or solid screws, or a combination thereof. In one embodiment, implant kit 300 comprises one or more bone plates, including any size or geometry of plate which may be necessary for the desired application. The one or more bone plates may accept locking screws, non-locking screws, or a combination thereof. In one embodiment, implant kit 300 comprises one or more plating screws. In one embodiment, implant kit 300 comprises one or more screwdrivers. The one or more screwdrivers may be cannulated, for use with cannulated screws. In one embodiment, the one or more screwdrivers may be solid, for use with solid screws and plating screws. In one embodiment, implant kit 300 comprises at least one cannulated screwdriver and at least one solid screwdriver. In one embodiment, implant kit 300 comprises one or more solid screw depth gauges, used to measure the length of plating screws using with or without a plate. In one embodiment, implant kit 300 comprises one or more cannulated depth gauges, used to measure the length of a cannulated screw utilizing a guide wire. In one embodiment, implant kit 300 comprises one or more preliminary fixation devices, including, but not limited to k-wires or olive wires, which may be placed through a bone plate to hold the plate in place prior to and during insertion of screws. In one embodiment, implant kit 300 comprises one or more guide wires. The guide wires are used to define and guide the trajectory of the cannulated screws and over which cannulated screws are inserted. In certain embodiments, the one or more guide wires comprise one or more visible markings to be used along with the countersink-depth gauge multi-tool device, as described elsewhere herein. In one embodiment, implant kit 300 comprises one or more drill bits, used to create a pilot hole for subsequent screw insertion. In certain embodiments, the one or more drill bits are cannulated for use with cannulated screws. In certain embodiments, the one or more drill bits are solid for use with solid screws. In one embodiment, implant kit 300 comprises at least one cannulated drill bit and one or more solid drill bit. In one embodiment, implant kit 300 comprises one or more drill guides. The one or more drill guides may be used to center a pilot hole within the bone plate when plating screws are used.

Accordingly, implant kit 300 may include some or all instrumentation required to place an implant construct for a particular surgical procedure. For example, in one embodiment, the kit comprises any combination of instrumentation for implantation of a cannulated screw, including a countersink-depth gauge multi-tool device, at least one cannulated screwdriver, at least one guide wire, at least one at least one cannulated drill bit and at least one cannulated screw. In another embodiment, implant kit 300 comprises any combination of instrumentation for implantation of a bone plate, including a countersink-depth gauge multi-tool device, at least one solid screwdriver, at least one preliminary fixation device, at least one solid drill bit, at least one drill guide and at least one bone plate. In another embodiment, implant kit 300 comprises any combination of instrumentation for implantation of both a bone plate and cannulated screw, where the sterile kit comprises a countersink-depth gauge multi-tool device, at least one cannulated screwdriver, at least one guide wire, at least one preliminary fixation device, at least one drill bit, at least one drill guide, at least one cannulated screw and at least one bone plate.

In certain embodiments, implant kit 300 is custom-configured with regard to size and geometry of the appropriate implant (e.g, bone plate, screw) being used. In one embodiment, the kit is configured for a specific size of screw or bone plate. In an alternative embodiment, the kit comprises instrumentation that may be necessary for various sizes of screws or bone plates, or multiple sized screws and/or bone plates.

In certain embodiments, the kit comprises instructional material. Instructional material may include a publication, a recording, a diagram, or any other medium of expression which can be used to communicate the usefulness of any of the devices or implant kits described herein. The instructional material may also include description of one or more steps to perform any of the methods described herein. The instructional material of the kit may, for example, be affixed to a package which contains one or more instruments which may be necessary for the desired procedure. Alternatively, the instructional material may be shipped separately from the package, or may be available electronically, such as accessible from the Internet, or any downloadable electronic document file format.

Still further, the present invention includes a method of providing a sterile implant kit as described herein. In certain embodiments, the method comprises receiving a request for one or more instruments for use in a surgical procedure. In certain embodiments, the method comprises a customized request for particular instrumentation. In certain embodiments, the method comprises a request for a standardized kit which would contain the one or more instruments. In certain instances the method comprises gathering the one or more instruments which were requested. In one embodiment, the method comprises processing the one or more instruments. For example, in one embodiment, the one or more instruments are sterilized. Sterilization of the one or more instruments may be conducted by any suitable method known in the art. In one embodiment, the method comprises packaging the one or more instruments. For example, the one or more instruments may be packaged in one or more sterile packages to form a sterile kit.

In one embodiment, the method further comprises providing the assembled kit to a user (i.e., surgeon, surgical staff member, medical assistant or provider, etc.) at a location for utilizing the instrumentation of the kit during the orthopedic procedure. In certain embodiments, the method comprises receiving one or more of the instruments not used during the procedure may be returned to the kit provider. In some embodiments, the returned instruments may result in a credit, discount, refund or other perk associated with the return of an unused instrument. In certain embodiments, the kit provider may use the returned instrumentation to form another kit.

It should be appreciated that the devices and the implant kits described herein may be used for a variety of surgical or non-surgical orthopedic procedures, including, but not limited to internal fixation of fractures, bone reconstruction, fusion of one or more bones or bone fragments, and the like. The procedures may be performed on any bone in the human or vertebrate body, including, but not limited to, scapula, olecranon, humerus, radius, ulna, pelvis, tibia, fibula, clavicle, vertebrae, femur, cranium, hand bones and foot bones. The internal fixation or reconstruction may include compression fractures, intra-articular and/or extra-articular factures, displaced fractures, osteotomies, non-unions and/or mal-unions. It should be understood that the present disclosure is not limited to a specific bone, fracture type, or surgical application.

The disclosures of each and every patent, patent application, and publication cited herein are hereby incorporated herein by reference in their entirety. While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

## Claims

1. A multi-purpose device for use in an orthopedic procedure, comprising:
an elongated body having a proximal end and a distal end and a length therebetween;
a countersink positioned at the distal end; and
a cannula extending from a distal end opening along at least a portion of the length of the elongated body;
wherein at least a portion of the cannula that is proximal to the countersink is visible through the outer surface of the elongated body.

2. The device of claim 1, wherein the countersink comprises one or more cutting flutes.

3. The device of claim 1, wherein the cannula has a diameter sized to receive a guide wire.

4. The device of claim 1, further comprising at least one graduation marking along the elongated body where at least a portion of the cannula is visible from the outer surface.

5. A system for use in an orthopedic procedure, comprising:
a tool having an elongated body comprising a proximal end and a distal end, wherein the distal end includes at least one fluted cutting edge and a distal end opening that leads into a cannula through an interior region of the elongated body, and wherein at least a portion of the cannula is visible from the outer surface of the elongated body in a cutaway region; and
a guide wire;
wherein the guide wire is sized to pass through the distal end opening and into the cannula of the tool, such that at least a portion of the guide wire is visible in the cutaway region.

6. The system of claim 5, wherein the tool comprises at least one graduation marking along the elongated body where at least a portion of the cannula is visible from the outer surface.

7. The system of claim 5, wherein the guide wire has at least one marking along its length, such that the at least one marking is identifiable when positioned in the cutaway region of the tool.

8. A method of determining the length of a screw to be inserted into a bone during an orthopedic procedure, comprising:
inserting the distal end of a guide wire having a predetermined length into bone, such that at least a portion of the proximal end of the guide wire extends outward from the bone surface;
advancing the proximal end of the guide wire through the distal end opening and into the cannula of the device of claim 1, such that the guide wire is inserted into at least a portion of the cannula;
positioning the distal end of the device in contact with the bone surface;
observing the location of proximal end of the guide wire within the exposed region of the cannula of the device;
measuring the length of the guide wire within the cannula;
calculating the length of the guide wire inserted into bone based on the difference between the predetermined length of the guide wire and the measured length of the guide wire within the cannula; and
determining the length of the screw based on the calculated length of the guide wire inserted into the bone.

9. The method of claim 8, wherein the device comprises at least one graduation marking along the elongated body where at least a portion of the cannula is visible from the outer surface, and wherein measuring the length of the guide wire within the cannula comprises identifying a graduation marking closest to the proximal end of the guide wire.

10. The method of claim 8, wherein the necessary length of the screw is equal to the calculated length of the guide wire inserted into the bone.

11. The method of claim 8, wherein the necessary length of the screw is equal to the calculated length of the guide wire minus the predetermined length of a region at the distal end of the guide wire.

12. A method of determining the length of a screw to be inserted into a bone during an orthopedic procedure, comprising:
inserting the distal end of a guide wire having at least one marking along its length into bone, such that at least a portion of the proximal end of the guide wire extends outward from the bone surface, wherein the at least one marking of the guide wire is indicative of the length of the guide wire from its distal end to the at least one marking;
advancing the proximal end of the guide wire through the distal end opening and into the cannula of the device of claim 1, such that the guide wire is inserted into at least a portion of the cannula;
positioning the distal end of the device in contact with the bone surface;
observing the location of the at least one marking of the guide wire within the exposed region of the cannula of the device;
measuring the length of the guide wire within the cannula;
calculating the length of the guide wire inserted into bone based on the difference between the length between the distal end of the guide wire and the at least one marking and the measured length of the guide wire within the cannula; and
determining the length of the screw based on the calculated length of the guide wire inserted into the bone.

13. The method of claim 12, wherein the device comprises at least one graduation marking along the elongated body where at least a portion of the cannula is visible from the outer surface, and wherein measuring the length of the guide wire within the cannula comprises identifying a graduation marking closest to the at least one marking of the guide wire.

14. A sterile kit comprising the device of claim 1,
wherein the kit further optionally comprises one or more instruments selected from the group consisting of an implantable screw, an implantable bone plate, a plating screw, a screwdriver, a screw depth gauge, a preliminary fixation device, a guide wire, a drill bit, and a drill guide, or
wherein optionally the one or more instruments are individually packaged in sterile packages, or
wherein optionally the kit comprises instructional material.

15. A method of providing a kit for use in a surgical procedure, comprising:
receiving a request for one or more instruments for use in a surgical procedure;
processing the one or more requested instruments;
packaging the one or more processed instruments into one or more sterile packages to form a sterile kit; and
providing the sterile kit to a user,
wherein optionally processing the one or more requested instruments comprises sterilizing the one or more requested instruments, or
wherein optionally the one or more instruments comprise a device comprising an elongated body having a proximal end and a distal end and a length therebetween; a countersink positioned at the distal end; and a cannula extending from a distal end opening along at least a portion of the length of the elongated body; wherein at least a portion of the cannula that is proximal to the countersink is visible through the outer surface of the elongated body.
